# EUROPÄISCHE PATENTSCHRIFT

(11) **EP 0 769 487 B1**
(45) Veröffentlichungstag und Bekanntmachung des Hinweises auf die Patenterteilung: **26.01.2000**
(21) Anmeldenummer: 96116054.6
(22) Anmeldetag: 08.10.1996
(51) Int. Cl.: C07C 59/68, G03C 7/00, C07C 59/70, C07C 51/367, C07C 69/712

(54) **Verfahren zur Herstellung von carboxylgruppenhaltigen Alkylarylethern**
Process for preparing carboxyl groups containing alkyl aryl ethers
Procédé de préparation d'éthers alkyle-aryle contenant des groupes carboxyliques

(30) Priorität: 20.10.1995 DE 19539073
(43) Veröffentlichungstag der Anmeldung: 23.04.1997
(73) Patentinhaber: BAYER AG, 51368 Leverkusen (DE)
(72) Erfinder: Antons, Stefan, Dr., 51373 Leverkusen (DE); Hammerschmidt, Erich, Dr., 51467 Bergisch Gladbach (DE); Blank, Heinz Ulrich, Dr., 51519 Odenthal (DE); Fiege, Helmut, Dr., 51373 Leverkusen (DE); Hartges, Heinz-Gerd, 51381 Leverkusen (DE)

(56) Entgegenhaltungen:
- EP-A- 0 115 007
- US-A- 2 511 231

## Beschreibung

Die Erfindung betrifft ein Verfahren zur Herstellung von carboxylgruppenhaltigen Alkylphenylethern durch eine spezielle Umsetzung von Phenolen mit carboxylgruppenhaltigen Alkylhalogeniden und basischen (Erd)-Alkaliverbindungen.

Aus US-A-2 511 231 ist die Herstellung von Farbfilmkupplern unter Verwendung von carboxylgruppenhaltigen Alkylarylethern sowie deren Säurechloriden bekannt.

In der Literatur ist die Herstellung von carboxylgruppenhaltigen Alkylphenylethern aus Phenolaten und carboxylgruppenhaltigen Alkylhalogeniden bereits auf unterschiedliche Weise realisiert worden. In US-A-25 11 231 und US-A-27 28 658 führt die Umsetzung von 2,4-Di-tert.-pentylphenol mit carboxylgruppenhaltigen Alkylhalogeniden wie Chloressigsäure oder α-Brom-n-buttersäure in wäßriger- bzw. wäßrig-alkoholischer Alkali jedoch zu geringen Ausbeuten und hohen Nebenproduktanteilen. Die Isolierung des gewünschten Ethers aus den dabei erhaltenen Reaktionsmischungen ist sehr aufwendig. So verursacht beispielsweise die in DD-A-261 277 vorgeschlagene Entfernung von nicht umgesetztem Restphenol als ein Kupplungsprodukt mit einem Diazoniumsalz zusätzliche Verfahrensschritte. Zur Vermeidung der teilweise durch die wäßrig-alkalische Natriumhydroxidlösung bedingten Nebenprodukte ist in DD 160 414 die Reaktion mit Alkalihydroxid in einem inerten aprotischen Lösungsmittel vorgeschlagen worden. Bei diesem Verfahren werden jedoch zusätzliche Lösungsmittel eingesetzt, die im Anschluß an die Reaktion getrennt aufgearbeitet und gegebenenfalls entsorgt werden müssen und somit zusätzlich aufwendige und belastende Verfahrensschritte erforderlich machen.

Die Aufgabe der vorliegenden Erfindung lag darin, ein verbessertes Verfahren zur Herstellung von carboxylgruppenhaltigen Alkylphenylethern bereitzustellen, bei dem die Nebenproduktbildung äußerst gering ist und bei dem der Einsatz zusätzlicher Lösungsmittel weitestgehend vermieden werden kann.

Es wurde nun ein Verfahren zur Herstellung von carboxylgruppenhaltigen Alkylphenylethern der Formel (I) 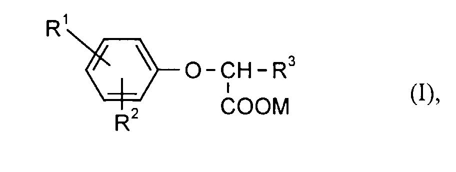 unter Einsatz von Phenolen der Formel (II) 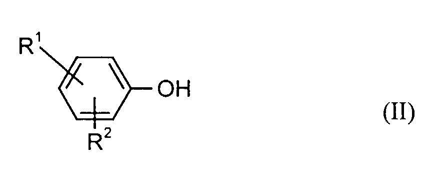 und carboxylgruppenhaltigen Alkylhalogeniden der Formel (III) 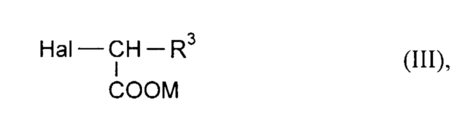 in Gegenwart von basischen (Erd)-Alkaliverbindungen gefunden,
wobei
- R¹ und R²: unabhängig voneinander für Wasserstoff, geradkettiges oder verzweigtes C₁-C₈-Alkyl, insbesondere Methyl, Ethyl, n-Propyl, Isopropyl, n-Butyl, sec.-Butyl, tert.-Butyl, n-Pentyl oder tert.-Pentyl, C₅-C₈-Cyclohexyl, insbesondere Cyclohexyl, C₆-C₁₀-Aryl-C₁-C₄-alkyl, insbesondere Benzyl, oder Ethylphenyl, C₆-C₁₀-Aryl, insbesondere Phenyl, Hydroxy, C₁-C₄-Alkoxy, C₆-C₁₀-Aryloxy oder Halogen, insbesondere Cl, Br oder J stehen,
- R³: Wasserstoff, geradkettiges oder verzweigtes C₁-C₈-Alkyl, insbesondere Methyl, Ethyl, n-Propyl, Isopropyl, n-Butyl, C₅-C₈-Cycloalkyl, insbesondere Cyclopentyl oder Cyclohexyl, C₆-C₁₀-Aryl-C₁-C₈-alkyl, insbesondere Benzyl oder Ethylphenyl, COOM, worin M = Na, Li, K oder C₁-C₄-Alkyl bedeutet,
- M: Na, Li, K oder C₁-C₄-Alkyl bedeutet, und
- Hal: für Cl, Br, F oder J, insbesondere für Br steht,
welches dadurch gekennzeichnet ist, daß die Umsetzung in der Schmelze des Phenols der Formel II erfolgt.

Bevorzugte basische (Erd)-Alkaliverbindungen sind solche, die mit den Phenolen der Formel II unter Bildung des entsprechenden Phenolats und Wasser oder eines aliphatischen C₁-C₄-Alkohols zu reagieren vermögen.

Besonders bevorzugte basische (Erd)-Alkaliverbindungen sind anorganische Alkaliverbindungen wie NaOH, Na₂CO₃, NaHCO₃, K₂CO₃, Li₂CO₃, KOH oder KHCO₃ und aliphatische C₁-C₄-Alkylalkoholate des Lithiums, Natriums oder Kaliums, insbesondere des Natriums, oder Mischungen davon. Die basischen (Erd)-Alkaliverbindungen können ohne Lösungs- oder Suspensionsmittel oder mit Wasser oder aliphatischen C₁-C₄-Alkoholen als Lösungs- oder Suspensionsmitteln eingesetzt werden. Sofern kein Lösungs- oder Suspensionsmittel verwendet wird, wird die (Erd)Alkaliverbindung vorzugsweise als Feststoff eingesetzt. Vorzugsweise werden die anorganischen Alkaliverbindungen fest oder insbesondere in Form ihren wäßrigen Suspensionen oder Lösungen eingesetzt, wobei letztere bevorzugt sind und im allgemeinen 5 bis 70 gew.-%ig, vorzugsweise gesättigt, sind.

Die Alkalialkoholate, insbesondere Alkalimethanolat oder -ethanolat, werden vorzugsweise in Lösungen des dem Alkoholat zugrunde liegenden Alkohols eingesetzt. Bevorzugt sind diese Lösungen 5 bis 70 gew.-%ig, vorzugsweise gesättigt.

Das erfindungsgemäße Verfahren erfolgt vorzugsweise bei oder oberhalb der Schmelztemperatur des eingesetzten Phenols der Formel II bei Normaldruck, besonders bevorzugt bei einer Temperatur von 50 bis 200°C, insbesonderc bei 80°C bis 150°C. Im allgemeinen erfolgt die Umsetzung bei Normaldruck, sie kann aber auch bei vermindertem Druck, insbesondere bei 20 bis 700 mbar oder bei erhöhtem Druck, insbesondere bei 1 bis 10 bar erfolgen.

Ganz besonders bevorzugt erfolgt die Umsetzung bei Temperaturen, die oberhalb der Siedetemperatur bei gegebenem Druck des bei der Bildung des entsprechenden Phenolats entstehenden Wassers oder Reaktionsalkohols sowie des gegebenenfalls für die basische (Erd)-Alkaliverbindung verwendeten Lösungs- oder Suspensionsmittel liegen.

Das bei der Bildung des Phenolats aus dem Phenol der Formel II vorzugsweise entstehende Reaktionswasser oder der entstehende Reaktionsalkohol wird zusammen mit den aliphatischen C₁-C₄-Alkohol- bzw. Wasseranteilen des gegebenenfalls für die basische (Erd)-Alkaliverbindung verwendeten Lösungs- oder Suspensionsmittels, vorzugsweise während der Umsetzung, besonders bevorzugt destillativ entfernt. Nach Beendigung der Reaktion ist der Anteil an Wasser und aliphatischen C₁-C₄-Alkoholen an der Reaktionsmischung vorzugsweise kleiner oder gleich 1 Gew.-%.

Zu den carboxylgruppenhaltigen Alkylphenylethern der Formel I gehören die Salze des Natriums, Lithiums oder Kaliums sowie die C₁-C₄-Alkylester, wie Methyl, Ethyl, n-Propyl, n-Butyl sowie die verzweigten Alkylester.

Zu den carboxylgruppenhaltigen Alkylhalogeniden der Formel III gehören entsprechend die Alkalisalze des Natriums, Lithiums oder Kaliums sowie die C₁-C₄-Alkylester.

Besonders bevorzugt werden die carboxylgruppenhaltigen Alkylphenylether mit einer bestimmten Bedeutung für M in Formel I unter Verwendung der carboxylgruppenhaltigen Alkylhalogenide mit der gleichen Bedeutung für M in Formel III hergestellt.

Für den Fall, daß das carboxylgruppenhaltige Alkylhalogenid in Form der freien Säure eingesetzt werden sollte, wird die Menge an (Erd)-Alkaliverbindung zweckmässigerweise um die Menge erhöht, die zur Neutralisation der Säure zum Carboxylat erforderlich ist.

Die molaren Verhältnisse der Reaktanden bei der Umsetzung nach dem erfindungsgemäßen Verfahren können im allgemeinen breit variiert werden. Das molare Verhältnis des Phenols der Formel II zu basischer (Erd)-Alkaliverbindung wird vorzugsweise so gewählt, daß eine vollständige Phenolatbildung des Phenols möglich ist. Das Verhältnis beträgt vorzugsweise 1:0,9 bis 0,9:1 pro zu verethemder aromatischer Hydroxygruppe. Es kann selbstverständlich auch darüber hinaus variiert werden. Das molare Verhältnis von Phenol und Alkylhalogenid beträgt vorzugsweise 1:1 bis 1:1,5, insbesondere 1:1 bis 1:1,1 pro zu verethernder aromatischer Hydroxygruppe. Auch hierbei kann das Verhältnis selbstverständlich breiter variiert werden.

Das erfindungsgemäße Verfahren wird vorzugsweise so durchgeführt, daß die basische (Erd)-Alkaliverbindung und das carboxylgruppenhaltige Alkylhalogenid mittels separater Zuleitungen bzw. Dosiereinrichtungen der Schmelze des Phenols der Formel I zugegeben werden. Dabei kann die Zugabe der beiden Komponenten nacheinander oder gleichzeitig erfolgen.

In einer besonders bevorzugten Ausführungsform des erfindungsgemäßen Verfahrens wird das carboxylgruppenhaltige Alkylhalogenid vollständig vor, teilweise vor oder gleichzeitig mit der basischen (Erd)-Alkaliverbindung zugegeben.

Ganz besonders bevorzugt ist die gleichzeitige Zugabe.

Bei der gleichzeitigen Zugabe kann es durchaus zu Zugabeschwankungen von dem gewünschten stöchiometrischen 1:1-Verhältnis der beiden Komponenten während der Zugabe kommen, ohne das dies dem Verständnis von einer gleichzeitigen Zugabe, wie sie im Rahmen dieser Anmeldung verstanden wird, schadet. Dabei sind Abweichungen von dem stöchiometrischen 1:1-Verhältnis von 1:1.05 bis 1.05:1 während der Zugabe durchaus normal. Etwaige Überschüsse einer Komponente werden bei einer gleichzeitigen Zugabe vorzugsweise im Anschluß zugegeben.

Für das erfindungsgemäße Verfahren einsetzbare Phenole der allgemeinen Formel (II) sind beispielsweise: 2-tert.-Butylphenol, 4-tert.-Butylphenol, 2,4-Di-tert.-butylphenol, 2-tert.-Pentylphenol, 4-tert.-Pentylphenol, 2,4-Di-tert.-pentylphenol, 2-tert.-Hexylphenol, 4-tert.-Hexylphenol, 2,4-Di-tert.-hexylphenol, 2-Chlor-4-tert.-amyl-phenol, 2-tert.-Butyl-hydrochinon.

Für das erfindungsgemäße Verfahren einsetzbare carboxylgruppenhaltige Alkylhalogenide der allgemeinen Formel (III) sind beispielsweise Chloressigsäuremethylester, Chloressigsäureethylester, Bromessigsäuremethylester, α-Brompropionsäureethylester, β-Brompropionsäureethylester, α-Brombuttersäureethylester, α-Bromisobuttersäureethylester, γ-Brombuttersäureethylester α-Bromisobuttersäureethylester.

Es kann auch vorteilhaft sein, zur Herstellung enantiomerer carboxylgruppenhaltiger Alkylphenylether die entsprechenden carboxylhaltigen Alkylhalogenide bereits in enantiomerer Form einzusetzen.

Carboxylgruppenhaltige Alkylarylether können nicht nur aus den Salzen der Carbonsäurealkylhalogenide sondern auch aus deren Estern hergestellt werden. Die Verseifung erfolgt vorzugsweise im Anschluß an die Reaktion.

Die nach beendeter Umsetzung vorliegenden (Erd)-Alkalihalogenide werden vorzugsweise nach dem Herabsetzen der Reaktionstemperatur mit der erforderlichen Menge an Wasser aus der erhaltenen Produktmischung ausgewaschen. Für den Fall, daß ein carbonsäuregruppenhaltiger Alkylphenylether in Form seiner freien Säure isoliert werden soll, wird zur Wäsche eine Säure, vorzugsweise eine wäßrige Mineralsäure wie beispielsweise HCl, H₂SO₄ usw. eingesetzt. Die nach dem erfindungsgemäßen Verfahren hergestellten Verbindungen sind von sehr hoher Reinheit und werden in sehr guten Ausbeuten, vorzugsweise >95 % der Theorie, erhalten. Der Vorteil des erfindungsgemäßen Verfahrens liegt weiterhin darin, daß auf ein zusätzliches Lösungsmittel während der Reaktion verzichtet werden kann.

Verwendet man estergruppenhaltige Alkylhalogenide, so kann beispielsweise durch Verwendung einer alkoholischen Alkoholat Lösung oder Suspension eines Alkohols, der verschieden ist von dem der Estergruppe zugrunde liegenden, eine teilweise oder vollständige Umesterung erfolgen.

Die bei dem erfindungsgemäßen Verfahren erhältlichen carboxylgruppenhaltigen Alkylphenylether der Formel I, insbesondere deren alkalische Salze oder deren C₁-C₄-Alkylester, werden vorzugseise durch Umsetzung mit Thionylchlorid oder COCl₂ oder anderen Chlorierungsmitteln in ihre Säurechloride übergeführt. Diese werden bei der Herstellung von Farbfilmkupplern eingesetzt. Die nach dem erfindungsgemäßen Verfahren erhaltenen carboxylgruppenhaltigen Alkylphenylether können beispielsweise zur Herstellung von Farbfilmkupplern wie sie in US-A-2 511 231 oder US-A-5 262 292 beschrieben sind oder als Zwischenprodukte für Pflanzenschutzmittel oder für Pharmazeutika verwendet werden.

### Beispiele

Abkürzungen: Me = Methyl, Et = Ethyl

### Beispiel 1 Darstellung von 2-(2,4-Di-tert.-butylphenoxy)capronsäureestergemisch

Unter Stickstoff wurden 206 g (1 Mol) 2,4-Di-tert.-butylphenol geschmolzen und auf 140°C erhitzt. Innerhalb von 1 Stunde wurden 185 g (1,03 Mol) einer 30 %igen NaOMe Lösung in MeOH und 245 g (1,1 Mol) 2-Bromcapronsäureethylester simultan zudosiert. Während der Zugabe wurde eine Mischung aus MeOH und EtOH abdestilliert. Nach der Zugabe war die Reaktion beendet. Es wurde auf 90°C abgekühlt und 250 ml Wasser zugesetzt. Nach Phasentrennung und Wäsche mit 200 ml Wasser verblieben 350 g eines viskosen gelben Öls der folgenden Zusammensetzung (0,8 % Bromcapronsäureethylester, 0,7 % Restphenol, 68 % 2-(2,4-Di-tert.-butylphenoxy)capronsäureethylester und 29 % 2-(2,4-Di-tert.-butylphenoxy)capronsäuremethylester). Dies entspricht einer Ausbeute von 98,8 % der Theorie, bezogen auf eingesetztes Phenol.

### Beispiel 2 Darstellung von 2-(2,4-Di-tert-butylphenoxy)capronsäureethylester

Unter Stickstoff wurden 206 g (1 Mol) 2,4-Di-tert.-butylphenol geschmolzen und auf 140°C erhitzt. Innerhalb von 1 Stunde werden 185 g (1,03 Mol) einer 30 %igen NaOMe-Lösung in MeOH und 245 g (1,1 Mol) 2-Bromcapronsäureethylester simultan zudosiert. Während der Zugabe wurde eine Mischung aus MeOH und EtOH abdestilliert. Nach der Zugabe war die Reaktion zum Estergemisch vollständig. Es wurde auf 80°C abgekühlt, 20 g NaOMe-Lösung zugesetzt und anschließend 750 ml Ethanol innerhalb von 4 Stunden zugesetzt. Bei einer Kopftemperatur von anfangs 74 bis 76°C destillierte ein Methanol/Ethanol-Gemisch ab. Am Ende wurde die Temperatur auf 90°C erhöht und reines Ethanol destillierte bei 78 bis 79°C über. Zur Isolierung wurden 250 ml Wasser zugesetzt. Nach Phasentrennung und erneuter Wäsche mit 200 ml Wasser verblieben 351 g eines viskosen gelben Öls der folgenden Zusammensetzung (0,3 % Bromcapronsäureethylester, 0,7 g Restphenol, 98 % 2-(2,4-Di-tert.-butylphenoxy)capronsäureethylester; 0,3 % 2-(2,4-Di-tert.-butylphenoxy)capronsäuremethylester. Dies entspricht einer Ausbeute von 98,8 % der Theorie, bezogen auf eingesetztes Phenol.

### Beispiel 3 Darstellung von 2-(2,4-Di-tert.-butylphenoxy)capronsäureethylester

Unter Stickstoff wurden 206 g (1 Mol) 2,4-Di-tert.-butylphenol geschmolzen und auf 140°C erhitzt. Innerhalb von 1 Stunde wurden 234 g (1,03 Mol) einer 30 %igen NaOEt-Lösung in EtOH und 245 g (1,1 Mol) 2-Bromcapronsäureethylester simultan zudosiert. Während der Zugabe wurde EtOH abdestilliert. Nach der Zugabe war die Reaktion beendet. Es wurde auf 90°C abgekühlt und 250 ml Wasser zugesetzt. Nach Phasentrennung und Wäsche mit 200 ml Wasser verblieben 347 g eines viskosen gelben Öls der folgenden Zusammensetzung (0,3 % Bromcapronsäureethylester, 0,5 % Restphenol, 98 % 2-(2,4-Di-tert.-butylphenoxy)capronsäureethylester). Dies entspricht einer Ausbeute von 98 % der Theorie, bezogen auf eingesetztes Phenol.

### Beispiel 4 Darstellung von 2-(2,4-Di-tert.-butylphenoxy)capronsäure

Es wurde wie im Beispiel 1 vorgegangen. Statt der Wasserzugabe wurden jedoch 240 g 25 %ige NaOH bei 90°C zugesetzt und 5 Stunden unter Rückfluß gekocht. Nach Phasentrennung wurde bis zu einer Kopftemperatur von 100°C andestilliert, um eventuelle Restalkohole abzudestillieren. Bei 90°C wurde mit 300 g 18 %iger HCI angesäuert und neutralgewaschen. Zur Entfernung von Restwasser wurde ein Vakuum von bis zu 20 mbar angelegt und 1 Stunde bei 90°C gerührt. Es resultierten 323 g schwach gelbgefärbter 2-(2,4-Di-tert.-butylphenoxy)capronsäure (Gehalte n. HPLC: 1,1 % Restphenol, 98,5 % 2-(2,4-Di-tert.-butylphenoxy)capronsäure). Dies entspricht einer Ausbeute von 99 % der Theorie, bezogen auf eingesetztes Phenol.

### Beispiel 5 Darstellung von 2-(2,4-Di-tert.-butylphenoxy)capronsäurechlorid

160 g der in Beispiel 4 hergestellten Carbonsäure wurden bei 80°C mit 200 g Thionylchlorid versetzt und 8 Stunden gerührt. Unter Vakuum wurde überschüssiges Thionylchlorid entfernt. Es verblieben 163 g des 96,5 %igen Säurechlorides.

### Beispiel 6 Darstellung von 2-(2,4-Di-tert.-butylphenoxy)capronsäureester

Es wurde wie in Beispiel 1 vorgegangen. Statt bei 140°C wurde die Reaktion bei 105 bis 110°C durchgeführt. Zur vollständigen Alkoholdestillation wurde bei 300 mbar gearbeitet.

Es wurden 348 g eines viskosen gelben Öls folgender Zusammensetzung erhalten (1,2 % Bromcapronsäureethylester, 0,8 % Restphenol, 73 % 2-(2,4-Di-tert.-butylphenoxy)capronsäureethylester und 25 % 2-(2,4-Di-tert.-butylphenoxy)capronsäuremethylester). Dies entspricht 99 % der Theorie.

### Beispiel 7 Darstellung von 2-(2,4-Di-tert.-pentylphenoxy)capronsäureester

Durchführung wie in Beispiel 1. Statt 2,4-Di-tert.-butylphenol wurden 234 g (1 Mol) 2,4-Di-tert.-pentylphenol eingesetzt.

Es resultierten 375 g eines Estergemisches der folgenden Zusammensetzung: 0,7 % Restphenol, 1,7 % 2-Bromcapronsäureethylester, 69 % 2-(2,4-Di-tert.-pentylphenoxy)capronsäureethylester, 27 % 2-(2,4-Di-tert.-pentylphenoxy)capronsäuremethylester. Dies entspricht 97 % der Theorie.

Die hieraus hergestellte Säure war 97,4 %ig rein (Schmp. 67°C).

### Beispiel 8 Darstellung von 2-(2,4-Di-tert.-pentylphenoxy)buttersäure

Durchführung analog Beispiel 1. Statt 2-Bromcapronsäureethylester wurden 210 g 2-Brombuttersäureethylester eingesetzt. Es resultieren 348 g eines Estergemisches. Dieses wurde wie beschrieben zu 320 g 2-(2,4-Di-tert.-pentylphenoxy)buttersäure (Schmp. 89°C; 96,8 %ig mit 1,2 % Restphenol; 97 % der Theorie) verseift.

### Beispiel 9 Darstellung von 2-(2,4-Di-pentylphenoxy)essigsäure

In analoger Weise wurden in 95,6 % der Theorie 96 %ige Säure (Schmp. 115 bis 120°C; 1,8 % Restphenol) aus Chloressigsäure und 2,4-Di-tert.-pentylphenol hergestellt.

### Beispiel 10 Darstellung von 2-(2-t-Butyl-4-tert.-pentylphenoxy)essigsäure

In analoger Weise wurden in 96,8 % der Theorie 97,8 %ige Säure (Schmp. 140 bis 144°C, 1,6 % Restphenol) aus 2-t-Butyl-4-tert.-pentylphenol hergestellt.

### Beispiel 11 Darstellung von 4-(4-tert.-Pentylphenoxy)-γ-buttersäure

In analoger Weise wurden in 92,6 % der Theorie 94 %ige Säure (2,8 % Restphenol) aus -Chlorbuttersäure und 4-tert.-Pentylphenol hergestellt.

### Beispiel 12 Darstellung von 4-tert.-Butylphenoxyessigsäure

In analoger Weise wurden in 93,8 % der Theorie 94 %ig reine Säure (Schmp. 93 bis 95°C, 1,4 % Restphenol) aus 4-tert.-Butylphenol und Chloressigsäuremethylester hergestellt.

### Beispiel 13 Darstellung von 2-(3-tert.-Butyl-4-hydroxyphenoxy)myristinsäure

In analoger Weise wie in Beispiel 1 wurden in 91 % d. Th. 92 %ige Säure (3,1 % Resthydrochinon) aus 2-tert.-Butylhydrochinon und α-Brommyristinsäureethylester erhalten.

### Beispiel 14 Darstellung von 2-(2-Chlor-4-cyclohexylphenoxymyristinsäure

In analoger Weise wie in Beispiel 1 wurden 92 % d. Th. 93,4 %ige Säure erhalten. Restgehalt 1,05 % 2-Chlor-4-cyclohexylphenol aus 2-Chlor-4-cyclohexylphenol und α-Brommyristinsäureethylester.

## Patentansprüche

1. Verfahren zur Herstellung von carboxylgruppenhaltigen Alkylphenylethem der Formel (I) 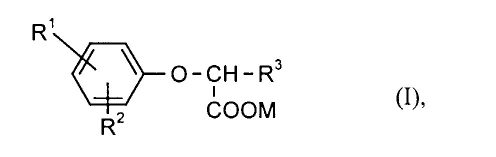 durch Umsetzung von Phenolen der Formel (II) 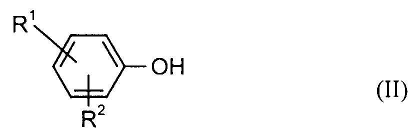 mit carboxylgruppenhaltigen Alkylhalogeniden der Formel (III) 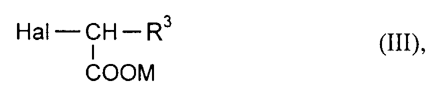 in Gegenwart von basischen (Erd)-Alkaliverbindungen,
wobei
R¹ und R² unabhängig voneinander für Wasserstoff, geradkettiges oder verzweigtes C₁-C₈-Alkyl, C₅-C₈-Cyclohexyl, C₆-C₁₀-Aryl-C₁-C₄-alkyl, C₆-C₁₀-Aryl, Hydroxy, C₁-C₄-Alkoxy, C₆-C₁₀-Aryloxy oder Halogen stehen,
R³ Wasserstoff, geradkettiges oder verzweigtes C₁-C₈-Alkyl, C₅-C₈-Cycloalkyl, C₆-C₁₀-Aryl-C₁-C₈-alkyl oder COOM bedeutet,
M Na, Li, K oder C₁-C₄-Alkyl bedeutet, und
Hal für Cl, Br, F oder J steht,
dadurch gekennzeichnet, daß die Umsetzung in der Schmelze des Phenols der Formel II erfolgt.

2. Verfahren gemäß Anspruch 1, dadurch gekennzeichnet, daß die basische (Erd)-Alkaliverbindung mit dem Phenol der Formel II unter Bildung des entsprechenden Phenolats und Wasser oder eines aliphatischen C₁-C₄-Alkohols zu reagieren vermag.

3. Verfahren gemäß Anspruch 1, dadurch gekennzeichnet, daß als basische (Erd)-Alkaliverbindung, NaOH, Na₂CO₃, K₂CO₃, Li₂CO₃, NaHCO₃, LiOH, KOH oder KHCO₃ oder ein aliphatisches C₁-C₄-Alkalialkoholat von Na, Li oder K, verwendet wird.

4. Verfahren gemäß Anspruch 2, dadurch gekennzeichnet, daß das bei der Bildung des Phenolats entstehende Wasser oder der entstehende Alkohol zusammen mit dem gegebenenfalls verwendeten Lösungs- oder Suspensionsmittel für die basische (Erd)-Alkaliverbindung während der Umsetzung entfernt wird.

5. Verfahren gemäß Anspruch 1, dadurch gekennzeichnet, daß das carboxylgruppenhaltige Alkylhalogenid der Formel III vollständig vor, teilweise vor oder gleichzeitig mit der (Erd)-Alkaliverbindung zu der Schmelze des Phenols der Formel II gegeben wird.

6. Verfahren gemäß Anspruch 1, dadurch gekennzeichnet, daß als Phenol der Formel II 2-tert.-Butylphenol, 4-tert.-Butylphenol, 2,4-Di-tert.-butylphenol, 2-tert.-Pentylphenol, 4-tert.-Pentylphenol, 2,4-Di-tert.-pentylphenol, 2-tert.-Hexylphenol, 4-tert.-Hexylphenol, 2,4-Di-tert.-hexylphenol, 2-Chlor-4-tert.-amyl-phenol, 2-tert.-Butyl-hydrochinon, und als caboxylgruppenhaltiges Alkylhalogenid der Formel III Chloressigsäuremethylester, Chloressigsäuremethylester, Chloressigsäureethylester, Bromessigsäuremethylester, α-Brompropionsäureethylester, α-Brombuttersäureethylester, α-Bromisobuttersäureethylester, γ-Brombuttersäureethylester oder α-Bromisobuttersäureethylester verwendet wird.

## Claims

1. Process for the preparation of alkyl phenyl ethers containing carboxyl groups, of the formula (I) 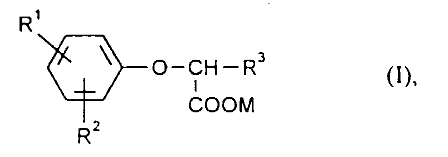 by reacting phenols of the formula (II) 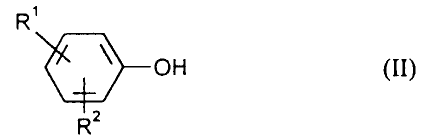 with alkyl halides containing carboxyl groups, of the formula (III) 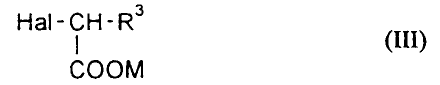 in the presence of basic alkali metal or alkaline earth metal compounds,
wherein
R¹ and R² independently of one another represent hydrogen, straight-chain or branched C₁-C₈-alkyl, C₅-C₈-cyclohexyl, C₆-C₁₀-aryl-C₁-C₄-alkyl, C₆-C₁₀-aryl, hydroxyl, C₁-C₄-alkoxy, C₆-C₁₀-aryloxy or halogen,
R3 represents hydrogen, straight-chain or branched C₁-C₈-alkyl, C₅-C₈-cycloalkyl, C₆-C₁₀-aryl-C₁-C₈-alkyl, or COOM,
M denotes Na, Li, K or C₁-C₄-alkyl and
Hal represents Cl, Br, F or I,
characterized in that the reaction is carried out in the melt of the phenol of the formula II.

2. Process according to Claim 1, characterized in that the basic alkali metal or alkaline earth metal compound is capable of reacting with the phenol of the formula II to form the corresponding phenolate and water or an aliphatic C1-C4-alcohol.

3. Process according to Claim 1, characterized in that NaOH, Na₂CO₃, K₂CO₃, Li₂CO₃, NaHCO₃, LiOH, KOH or KHCO₃ or an aliphatic alkali metal C₁-C₄-alcoholate of Na, Li or K is used as the basic alkali metal or alkaline earth metal compound.

4. Process according to Claim 2, characterized in that the water formed during the formation of the phenolate or the alcohol formed is removed during the reaction together with the solvent or suspending agent used, where appropriate, for the basic alkali metal or alkaline earth metal compound.

5. Process according to Claim 1, characterized in that the alkyl halide containing carboxyl groups of the formula III is added to the melt of the phenol of the formula II entirely before, partly before or simultaneously with the alkali metal or alkaline earth metal compound.

6. Process according to Claim 1, characterized in that 2-tert-butylphenol, 4-tert-butylphenol, 2,4-di-tert-butylphenol, 2-tert-pentylphenol, 4-tert-pentylphenol, 2,4-di-tert-pentylphenol, 2-tert-hexylphenol, 4-tert-hexylphenol, 2,4-di-tert-hexylphenol, 2-chloro-4-tert-amyl-phenol or 2-tert-butyl-hydroquinone is used as the phenol of the formula II and methyl chloroacetate, ethyl chloroacetate, methyl bromoacetate, ethyl α-bromopropionate, ethyl α-bromobutyrate, ethyl α-bromoisobutyrate, ethyl γ-bromobutyrate or ethyl α-bromoisobutyrate is used as the alkyl halide containing carboxyl groups of the formula III.

## Revendications

1. Procédé pour la préparation d'alkylphényléthers carboxylés répondant à la formule (I) 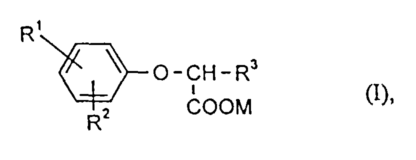 par la mise en réaction de phénols répondant à la formule (II) 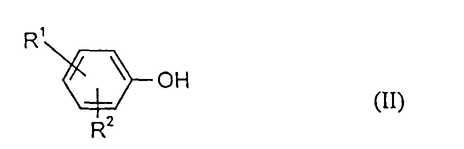 avec des halogénures d'alkyle carboxylés répondant à la formule (III) 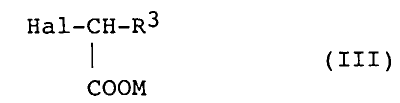 en présence de composés basiques de métaux alcalins (alcalino-terreux)
dans lequel
R¹ et R² représentent, indépendamment l'un de l'autre, un atome d'hydrogène, un groupe alkyle en C₁-C₈ à chaîne droite ou ramifiée, un groupe cyclohexyle en C₅-C_{8,} un groupe aryl(en C₆-C₁₀)alkyle en C₁-C₄, un groupe aryle en C₆-C₁₀, un groupe hydroxyle, un groupe alcoxy en C₁-C₄, un groupe aryl(en C₆-C₁₀)oxy ou un atome d'halogène,
R³ représente un atome d'hydrogène, un groupe alkyle en C₁-C₈ à chaîne droite ou ramifiée, un groupe cycloalkyle en C₅-C_{8,} un groupe aryl(en C₆-C₁₀)-alkyle en C₁-C₈ ou un groupe COOM,
M représente un atome de sodium, un atome de lithium, un atome de potassium ou un groupe alkyle en C₁-C_{4,} et
Hal représente un atome de chlore, un atome de brome, un atome de fluor ou un atome d'iode,
caractérisé en ce que la mise en réaction a lieu dans la masse fondue du phénol répondant à la formule II.

2. Procédé selon la revendication 1, caractérisé en ce que le composé basique de métal alcalin (alcalino-terreux) est à même de réagir avec le phénol répondant à la formule II en formant le phénolate correspondant et de l'eau ou un alcool aliphatique en C₁-C₄.

3. Procédé selon la revendication 1, caractérisé en ce qu'on utilise à titre de composé basique de métal alcalin (alcalino-terreux) du NaOH, du Na₂CO₃, du K₂CO₃, du Li₂CO₃, du NaHCO₃, du LiOH, du KOH ou du KHCO₃, ou encore un alcoolate alcalin aliphatique en C₁-C₄ du sodium, du lithium ou du potassium.

4. Procédé selon la revendication 2, caractérisé en ce que l'eau que l'on obtient lors de la formation du phénolate ou l'alcool que l'on obtient sont éliminés au cours de la mise en réaction de manière conjointe avec le solvant ou l'agent de mise en suspension utilisé le cas échéant pour le composé basique de métal alcalin (alcalino-terreux).

5. Procédé selon la revendication 1, caractérisé en ce que l'halogénure d'alkyle carboxylé répondant à la formule III est ajouté à la masse fondue du phénol répondant à la formule II complètement avant, en partie avant ou simultanément avec le composé de métal alcalin (alcalino-terreux).

6. Procédé selon la revendication 1, caractérisé en ce qu'on utilise, à titre de phénol répondant à la formule II, le 2-tert.-butylphénol, le 4-tert.-butylphénol, le 2,4-di-tert.-butylphénol, le 2-tert.-pentylphénol, le 4-tert.-pentylphénol, le 2,4-di-tert.-pentylphénol, le 2-tert.-hexylphénol, le 4-tert.-hexylphénol, le 2,4-di-tert.-hexylphénol, le 2-chloro-4-tert.-amylphénol, la 2-tert.-butylhydroquinone et, à titre d'halogénure d'alkyle carboxylé répondant à la formule III, on utilise l'ester méthylique de l'acide chloracétique, l'ester éthylique de l'acide chloracétique, l'ester méthylique de l'acide bromacétique, l'ester éthylique de l'acide α-bromopropionique, l'ester éthylique de l'acide α-bromobutyrique, l'ester éthylique de l'acide α-bromo-isobutyrique, l'ester éthylique de l'acide γ-bromobutyrique ou l'ester éthylique de l'acide α-bromoisobutyrique.
